# EUROPEAN PATENT SPECIFICATION

(11) **EP 2 276 145 B1**
(45) Date of publication and mention of the grant of the patent: **11.01.2017**
(21) Application number: 09738732.8
(22) Date of filing: 22.04.2009
(51) Int. Cl.: H02J 7/02, A61B 1/00, H02J 5/00

(54) **WIRELESS POWER SUPPLY SYSTEM**
DRAHTLOSES STROMVERSORGUNGSSYSTEM
SYSTEME D'ALIMENTATION ELECTRIQUE SANS FIL

(30) Priority: 02.05.2008 JP 2008120605
(43) Date of publication of application: 19.01.2011
(73) Proprietor: Olympus Corporation, Tokyo 192-8507 (JP)
(72) Inventor: IWAISAKO, Hiroshi, Tokyo 192-8507 (JP)
(74) Representative: Winter, Brandl, Fürniss, Hübner, Röss, Kaiser, Polte - Partnerschaft mbB
(86) International application number: PCT/JP2009/057974
(87) International publication number: WO 2009/133795

(56) References cited:
- WO-A1-92/17929
- WO-A1-2005/093927
- WO-A2-2005/106901
- DE-A1- 10 158 794
- JP-A- 9 308 140
- JP-A- 2001 231 186
- JP-A- 2003 309 971
- JP-A- 2004 159 456
- JP-A- 2004 159 456
- JP-A- 2008 283 789
- JP-A- 2008 283 790
- JP-A- 2008 283 791
- JP-A- 2008 283 792

## Description

### Technical Field

The present invention relates to a wireless power feeding system which wirelessly supplies electric power from outside of a body to an in-vivo information acquiring device, such as a capsule endoscope, that operates inside of the body.

### Background Art

Wireless power feeding system that contactlessly supplies electric power from outside of a body to a certain device such as a capsule endoscope operating in a subject's body has been proposed, such as the one discloses in Japanese Patent Application Laid-Open Publication No. 2004-159456 in which an electric current is passed through primary coils provided in the wireless power feeding system to induce electrical energy in a secondary coil provided in the device.

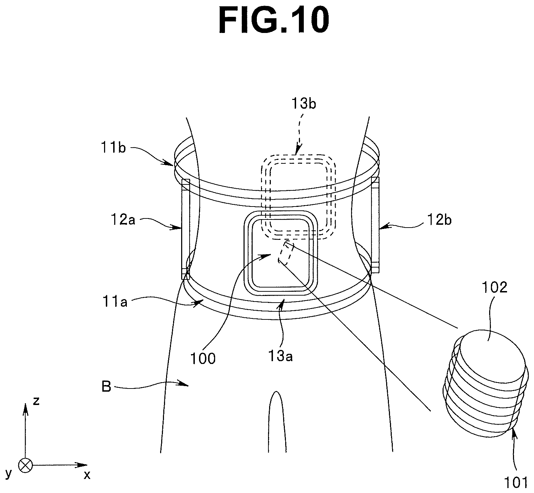

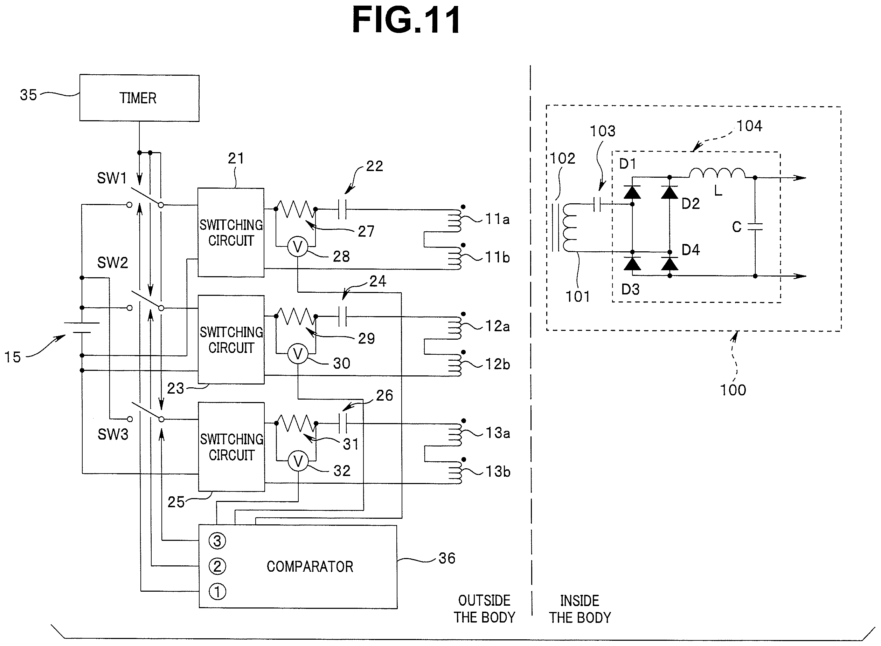

The configuration of the primary coils provided in the wireless power feeding system in the proposal described in the Japanese Patent Application Laid-Open Publication No. 2004-159456 will be briefly described below with reference to Figs. 10 and 11.

Fig. 10 illustrates the primary coil configuration of the existing wireless power feeding system. Illustrated in Fig. 10 is the configuration of the wireless power feeding system for capsule endoscope, in which X-, Y-, and Z-axis primary coils are attached to the body of a subject B and electric power is wirelessly supplied to the capsule endoscope which is a small medical device in a body cavity of the subject B.

In Fig. 10, Helmholtz power transmission coil pairs are arranged in a three-dimensional Cartesian coordinate system around the body of a subject B, that is, in the X-, Y-, and Z-axis directions that are orthogonal to each other. Power transmission coils 12a and 12b are placed along the X-axis direction; power transmission coils 13a and 13b are placed along the Y-axis direction; power transmission coils 11a and 11b are placed along the Z-axis direction.

Fig. 11 illustrates a circuit configuration of power transmission coils in the existing wireless power feeding system. When electric power is supplied to a capsule endoscope 100, power transmission coils 11a and 11b, 12a and 12b, and 13a and 13b are connected in pairs in series. The pairs of series-connected power transmission coils 11a and 11b, 12a and 12b, and 13a and 13b are connected to power-transmission-coil resonant capacitors 22, 24 and 26, respectively, in series to form resonance circuits as illustrated in Fig. 11.

The existing wireless power feeding system illustrated in Fig. 11 includes the resonance circuits described above, switching circuits 21, 23 and 25 which supply an AC voltage to the power transmission coils, a power supply unit 15 which supplies electric power to the switching circuits 21, 23 and 25, and switches SW1, SW2 and SW3 which select either supplying or removing electric power to the switching circuits 21, 23 and 25.

In the existing wireless power feeding system described above, a Helmholtz power transmission coil pair that can most efficiently supply electric power, which depends on the location and orientation of the capsule endoscope in the body of the subject B, is selected from among the three Helmholtz power transmission coil pairs and an AC volt is applied to that power transmission coil pair. In this way, electric power can be wirelessly supplied to the capsule endoscope with a high efficiency.

When an AC voltage is applied to power transmission coils to generate a magnetic field, generally a resonance circuit including coils and a capacitor is used in order to increase the efficiency of power supply. As has been described above, in the proposal described in Japanese Patent Application Laid-Open Publication No. 2004-159456, power transmission coils and a resonant capacitor are connected in series in order to increase the efficiency of power supply to each power transmission coil pair and a resonant circuit is used to drive a power transmission antenna.

However, when an AC current is applied to a desired one of the three power transmission coil pairs in the proposal described in Japanese Patent Application Laid-Open Publication No. 2004-159456, which is the basis for the preamble of the independent claims, an induced electromotive force is generated in the other two power transmission coil pairs to which the AC voltage is not being applied, because resonance frequencies of the three power transmission coil pairs are equal. That is, an electric current flows through one power transmission coil pair to which an AC current is applied and, in addition, the other two power transmission coil pairs in which the induced electromotive force is generated. As a result, the electric current flowing through the power transmission coil pairs becomes unstable, which in turn destabilizes the orientation of a magnetic field generated at the power transmission coil pairs.

That is, the proposal described in Japanese Patent Application Laid-Open Publication No. 2004-159456 has a problem that it is difficult to properly control electric energy being transmitted and the direction of power transmission and therefore the efficiency of power supply decreases.

The present invention has been made in light of these circumstances and an object of the present invention is to provide a wireless power feeding system that wirelessly supplies electric power from outside of a body and is capable of efficiently supplying electric power by stably controlling the magnitude of an electric current passing through a power transmission coil and appropriately controlling the intensity and orientation of a magnetic field generated by the power transmission coil.

The object is solved by the features of the independent claims. The dependent claims are directed to preferred embodiments of the invention.

### Brief Description of the Drawings

Fig. 1 is a schematic diagram illustrating a configuration of a wireless power feeding system according to a first embodiment of the present invention;
Fig. 2 is a schematic front view illustrating power transmission coils 43, 53 and 63 located around the body of a subject along three axes, viewed from the front of the subject;
Fig. 3 is a schematic cross-sectional view illustrating the power transmission coils 43, 53 and 63 located around the body of the subject along the three axes, viewed from above the subject;
Fig. 4 is a schematic diagram illustrating a configuration of a wireless power feeding system according to a second embodiment of the present invention;
Fig. 5 is a schematic diagram illustrating a configuration of a wireless power feeding system according to a third embodiment of the present invention;
Fig. 6 is a schematic diagram illustrating a configuration of a wireless power feeding system according to a fourth embodiment of the present invention;
Fig. 7 is a schematic diagram illustrating a configuration of a wireless power feeding system according to a fifth embodiment of the present invention;
Fig. 8 is a schematic diagram illustrating a configuration of a wireless power feeding system according to a sixth embodiment of the present invention;
Fig. 9 is a schematic diagram illustrating a variation of the wireless power feeding system according to the sixth embodiment of the present invention;
Fig. 10 is a diagram illustrating a primary coil configuration in an existing wireless power feeding system; and
Fig. 11 is a circuit diagram of primary coils in the existing wireless power feeding system.

### Best Mode for Carrying Out the Invention

Embodiments of the present invention will be described with reference to drawings.

### (First embodiment)

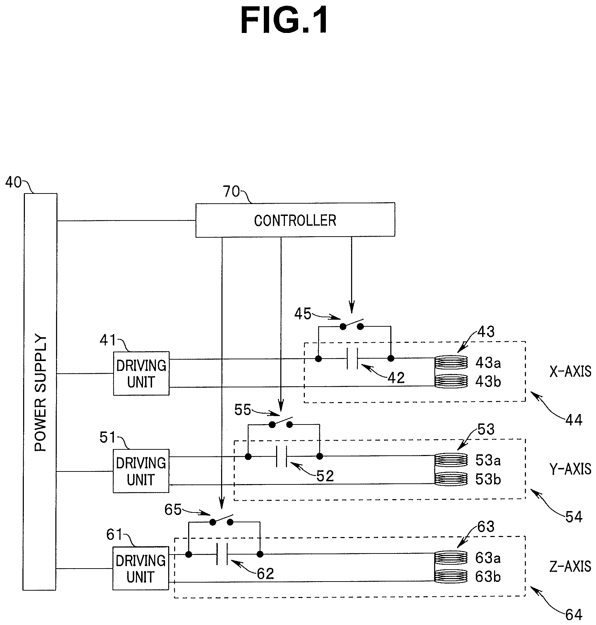

A configuration of a wireless power feeding system will be described first with reference to Fig. 1. Fig. 1 is a schematic diagram illustrating a configuration of a wireless power feeding system according to a first embodiment of the present invention.

The wireless power feeding system in Fig. 1 mainly includes three power transmission antennas 44, 54 and 64, driving units 41, 51 and 61 which drive the power transmission antennas 44, 54 and 64, a controller 70 which performs control to allow an electric current to flow through capacitors 42, 52, 62 or to flow without passing through the capacitors 42, 52, 62, and a power supply 40 which is electrically connected to the driving units 41, 51 and 61 and the controller 70 and supplies electric power to the driving units 41, 51 and 61 and the controller 70.

The power supply 40 is an alternating current (AC) power supply, an AC-to-direct-current (DC) conversion power supply, or a direct-current (DC) power supply, or the like. The driving units 41,51 and 61 are electrically connected to the power transmission antennas 44, 54 and 64, respectively, and apply a voltage outputted from the power supply 40 to the power transmission antennas 44, 54 and 64, respectively.

The power transmission antenna 44 is formed by a series resonance circuit in which the capacitor 42 and a power transmission coil 43 for X-axis drive are connected in series. The power transmission coil 43 includes coils 43a and 43b connected in a Helmholtz arrangement.

A switch 45 is connected to the ends of the capacitor 42 for controlling a resonant state of the power transmission antenna 44. Specifically, the switch 45 is connected with the capacitor 42 in parallel and is capable of turning on and off a current flow across the capacitor 42. When the switch 45 is turned off (open), a current flows to the power transmission coil 43 through the capacitor 42; when the switch 45 is turned on (in conduction), no current flows through the capacitor 42 but a current flows to the power transmission coil 43 through the switch 45.

Like the power transmission antenna 44, the power transmission antenna 54 is formed by a series resonance circuit in which the capacitor 52 and a power transmission coil 53 for Y-axis drive are connected in series. The power transmission coil 53 includes coils 53a and 53b connected in a Helmholtz arrangement.

A switch 55 is connected to the ends of the capacitor 52 for controlling a resonant state of the power transmission antenna 54. Specifically, the switch 55 is connected with the capacitor 52 in parallel and is capable of turning on and off a current flow across the capacitor 52. When the switch 55 is turned off (open), a current flows to the power transmission coil through the capacitor 52; when the switch 55 is turned on (in conduction), no current flows through the capacitor 52 but a current flows to the power transmission coil 53 through the switch 55.

Like the power transmission antennas 44 and 54, the power transmission antenna 64 is formed by a series resonance circuit in which the capacitor 62 and a power transmission coil 63 for Z-axis drive are connected in series. The power transmission coil 63 includes coils 63a and 63b connected in a Helmholtz arrangement.

A switch 65 is connected to the ends of the capacitor 62 for controlling a resonant state of the power transmission antenna 64. Specifically, the switch 65 is connected with the capacitor 62 in parallel and is capable of turning on and off a current flow across the capacitor 62. When the switch 65 is turned off (open), a current flows to the power transmission coil 63 through the capacitor 62; when the switch 65 is turned on (in conduction), no current flows through the capacitor 62 but a current flows to the power transmission coil 63 through the switch 65.

Each of the switches 45, 55 and 65 is turned on and off according to a control signal from the controller 70. The switches 45, 55 and 65 are not limited to switches with contacts and semiconductor switches; they may be any switches that are capable of switching between blocking and maintaining a current path.

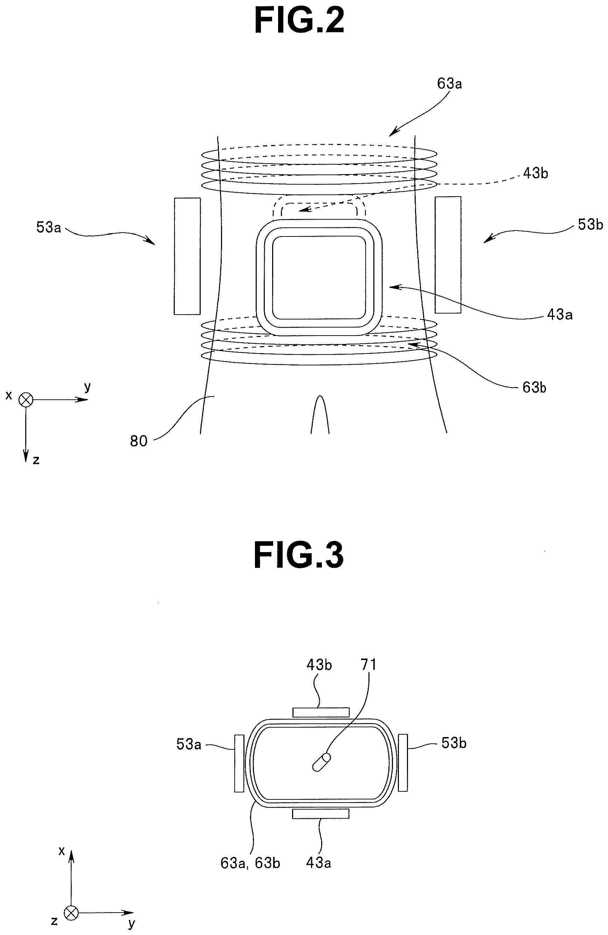

The power transmission coil 43 for X-axis drive, the power transmission coil 53 for Y-axis drive, and the power transmission coil 63 for Z-axis drive are located in a substantial three-dimensional Cartesian coordinate system around the body of a subject as illustrated in Figs. 2 and 3. Fig. 2 is a schematic front view illustrating the power transmission coils 43, 53 and 63 located around the body of the subject along the three axes, viewed from the front of the subject. Fig. 3 is a schematic cross-sectional view illustrating the power transmission coils 43, 53 and 63 located around the body of the subject along the three axes, viewed from above the subject.

Applying a high-frequency voltage to one of the X-, Y-, and Z-axis power transmission coils can cause the power transmission coil to generate an AC magnetic field to wirelessly supply electric power to a capsule endoscope 71, which is a in-vivo information acquiring device retained inside the body of a subject 80.

An operation of the wireless power feeding system configured as described above will be described below. An operation to apply an AC voltage only to the X-axis power transmission coil among the X-, Y-, and Z-axis power transmission coils illustrated in Figs. 2 and 3 to generate a magnetic field for the X-axis will be described first.

First, electric power is supplied from the power supply 40 to the driving unit 41. In the driving unit 41, the supplied electric power is converted to high-frequency power with the same frequency as a resonance frequency of the series resonance circuit made up of the capacitor 42 and the power transmission coil 43. The high-frequency power is applied by the driving unit 41 to the power transmission antenna 44 formed by the capacitor 42 and the power transmission coil 43 connected in series.

Since a magnetic field does not need to be generated at the power transmission coils 53 and 63, an AC current (high-frequency voltage) is not generated in the driving unit 51 and 61 connected to the power transmission coils 53 and 63.

The controller 70 sends out a control signal to turn off the switch 45 attached to the X-axis power transmission antenna 44. When the switch 45 is turned off, the power transmission antenna 44 can form a resonance circuit. Consequently, a magnetic field is generated at the power transmission coil 43 by the applied high-frequency power.

At the same time, the controller sends a control signal to turn on the switch 55 attached to the Y-axis power transmission antenna 54 and the switch 65 attached to the Z-axis power transmission antenna 64. When the switches 55 and 65 are turned on by the control, the power transmission antennas 54 and 64 become unable to form resonance circuits.

Accordingly, even if the Y-axis power transmission coil 53 and/or the Z-axis power transmission coil 63 is exposed to the magnetic field generated from the X-axis power transmission coil 43, generation of an induced electromotive force by the magnetic field can be sufficiently inhibited. Therefore a magnetic field can be generated only from the X-axis power transmission coil 43 with a desired intensity in a desired orientation.

An operation to apply an AC voltage only to the Y-axis to generate a magnetic field for the Y-axis will be described next. In this case, the X-axis components for generating the magnetic field for the X-axis in the above description can simply be replaced with the Y-axis components equivalent to those components. Specifically, the controller 70 sends out a control signal to turn off the switch 55 attached to the Y-axis power transmission antenna 54. At the same time, the controller 70 sends out a control signal to turn on the switch 45 attached to the X-axis power transmission antenna 44 and the switch 65 attached to the Z-axis power transmission antenna 64. Electric power supplied from the power supply 40 is converted to an AC voltage (high-frequency voltage) by the driving unit 51 and the high-frequency voltage is applied only to the Y-axis power transmission antenna 54.

With the operation described above, even it the X-axis power transmission coil 43 and/or the Z-axis power transmission coil 63 is exposed to the magnetic field generated from the Y-axis power transmission coil 53, generation of an induced electromotive force by the magnetic field can be sufficiently inhibited. Accordingly, a magnetic field can be generated only from the Y-axis power transmission coil 53 with a desired intensity in a desired orientation.

When an AC voltage is to be applied only to the Z-axis to generate a magnetic field for the Y-axis, an operation and control similar to those for the X and Y axes are performed. Specifically, the controller 70 sends out a control signal to turn off the switch 65 attached to the Z-axis power transmission antenna 64. At the same time, the controller 70 sends out a control signal to turn on the switch 45 attached to the X-axis power transmission antenna 44 and the switch 55 attached to the Y-axis power transmission antenna 54. Electric power supplied from the power supply 40 is converted to an AC voltage (high-frequency power) by the driving unit 61 and the high-frequency power is applied only to the Z-axis power transmission antenna 64.

With the operation described above, even if the X-axis power transmission coil 43 and/or the Y-axis power transmission coil 53 is exposed to the magnetic field generated from the Z-axis power transmission coil 63, generation of an induced electromotive force by the magnetic field can be sufficiently prevented. Accordingly, a magnetic field can be generated only from only the Z-axis power transmission coil 63 with a desired intensity in a desired orientation.

In this way, since the switches 45, 55 and 65 are provided at the power transmission antennas 44, 54 and 64 of the three axes and can be turned on and off by the controller 70 to place only a power transmission antenna of an axis from which a magnetic filed is to be generated in a resonant state and to place the other power transmission antennas of the other two axes in a nonresonant state in the wireless power feeding system of the present embodiment, the magnitude of an electric current to pass through each power transmission coil can be stably controlled and the intensity and orientation of the magnetic field generated from the power transmission coil can be properly controlled. Accordingly, electric power can be efficiently supplied to the in-vivo information acquiring device. Furthermore, an induced electromotive force can be inhibited from being generated between power transmission coils and therefore useless electric power is eliminated and energy savings can be achieved.

### (Second embodiment)

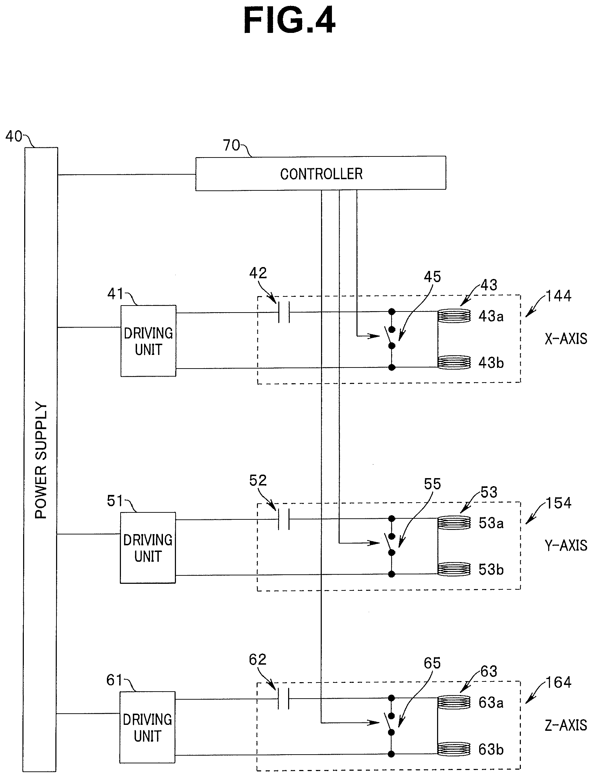

A wireless power feeding system according to a second embodiment of the present invention will be described with reference to Fig. 4 in detail. Fig. 4 is a schematic diagram illustrating a configuration of the wireless power feeding system according to the second embodiment of the present invention.

The configuration of the wireless power feeding system of the present embodiment is the same as that of the wireless power feeding system of the first embodiment described with reference to Fig. 1 with the only difference being the circuit configuration of power transmission antennas 144, 154 and 164 corresponding to X-, Y- and Z-axes, respectively. Therefore, only the circuit configuration of the power transmission antennas 144, 154 and 164 will be described here and the same components as those of the first embodiments are given the same reference symbols and description of the same components will be omitted.

The power transmission antennas 144, 154 and 164 of the X-, Y-, and Z-axes have the same circuit configuration. Therefore only the X-axis power transmission antenna 144 will be described here and description of the Y- and Z-axis power transmission antennas 154 and 164 will be omitted.

In the first embodiment, the switch 45 is connected to the ends of the capacitor 42 in the power transmission antenna 44 including the series resonance circuit in which the capacitor 42 and the power transmission coil 43 are connected in series. The second embodiment differs from the first embodiment in that a switch 45 is connected to the ends of a power transmission coil 43 in a power transmission antenna 144 including a series resonance circuit in which a capacitor 42 and a power transmission coil 43 are connected in series.

The switch 45 turns on and off the connection between the ends of the power transmission coil 43 according to a control signal sent from a controller 70. Similarly, switches 55 and 65 are connected to the ends of a Y-axis power transmission coil 53 and a Z-axis power transmission coil 63, respectively, as in the power transmission antenna 144.

Specifically, when a magnetic field is to be generated along the X-axis, the switch 45 is turned off. At the same time, the switches 55 and 65 for the Y- and Z-axes are turned on. By controlling and turning on and off the switches 45, 55 and 65 in this way, the X-axis power transmission antenna 144 is placed in a resonant state and the Y-axis power transmission antenna 154 and the Z-axis power transmission antenna 164 are placed in a nonresonant state. Accordingly, a magnetic field can be generated only from the X-axis power transmission antenna with a desired intensity in a desired orientation.

Control of turning on and off of the switches to generate a magnetic field for the Y axis and a magnetic field for the Z axis is similar to the control for the X-axis and can be achieved simply by replacing the components used for the X-axis with the components for the Y- and Z-axes, as in the first embodiment. Therefore description of operations for the Y- and Z-axes will be omitted.

In this way, since the switches 45, 55 and 65 are provided at the power transmission antennas 144, 154 and 164 of the three axes and can be turned on and off by the controller 70 to place only a power transmission antenna of an axis from which a magnetic filed is to be generated in a resonant state and to place the other power transmission antennas of the other two axes in a nonresonant state in the present embodiment, the magnitude of an electric current to pass through each power transmission coil can be stably controlled and the intensity and orientation of the magnetic field generated from the power transmission coil can be properly controlled. Accordingly, electric power can be efficiently supplied to the in-vivo information acquiring device. Furthermore, an induced electromotive force can be inhibited from being generated between power transmission coils and therefore useless electric power is eliminated and energy savings can be achieved.

### (Third embodiment)

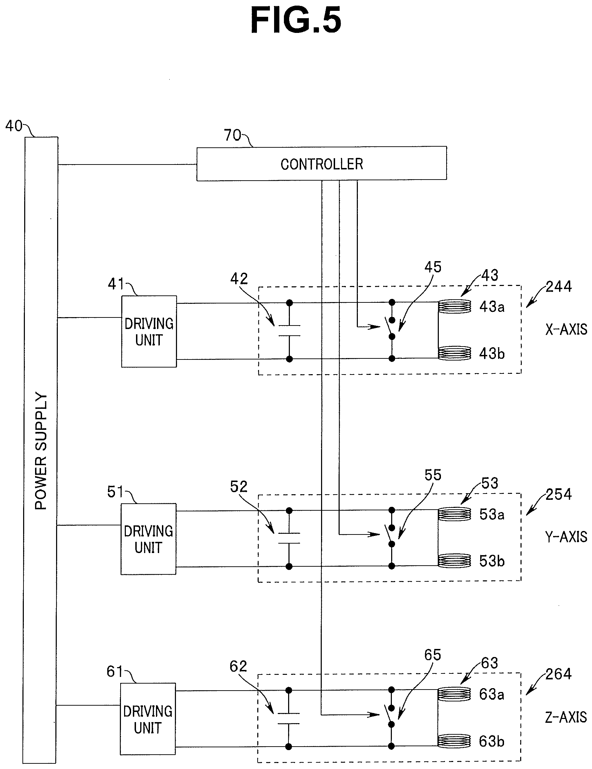

A wireless power feeding system according to a third embodiment of the present invention will be described below with reference to Fig. 5 in detail. Fig. 5 is a schematic diagram illustrating a configuration of the wireless power feeding system according to the third embodiment of the present invention.

The configuration of the wireless power feeding system of the present embodiment is the same as the wireless power feeding system of the first embodiment described with reference to Fig. 1 with the only difference being the circuit configuration of power transmission antennas 244, 254 and 264 corresponding to the X-, Y- and Z axes, respectively. Therefore, only the circuit configuration of the power transmission antennas 244, 254 and 264 will be described here and the same components as those of the first embodiment are given the same reference symbols and description of the same components will be omitted.

The X-, Y- and Z-axis power transmission antennas 244, 254 and 264 have the same circuit configuration. Therefore only the X-axis power transmission antenna 244 will be described here and description of the Y- and Z-axis power transmission antennas 254 and 264 will be omitted.

In the first embodiment, the switch 45 is connected to the ends of the capacitor 42 in the power transmission antenna 44 including a series resonance circuit in which the capacitor 42 and the power transmission coil 43 are connected in series. The present embodiment differs from the first embodiment in that a switch 45 is connected to the ends of a capacitor 42, that is, to the ends of a power transmission coil 43, in a power transmission antenna 244 including a parallel resonance circuit in which the capacitor 42 and the power transmission coil 43 are connected in parallel.

The switch 45 turns on and off the connection between the ends of power transmission coil 43 according to a control signal sent from a controller 70. Switches 55 and 65 are connected to the ends of a Y-axis power transmission coil 53 and a Z-axis power transmission coil 63, respectively, as in the power transmission antenna 244.

Specifically, when a magnetic field is to be generated along the X-axis, the switch 45 is turned off. At the same time, the switches 55 and 65 for the Y- and Z-axes are turned on. By controlling and turning on and off the switches 45, 55 and 65 in this way, the X-axis power transmission antenna 244 is placed in a resonant state and the Y-axis power transmission antenna 254 and the Z-axis power transmission antenna 264 are placed in a nonresonant state. Accordingly, a magnetic field can be generated only from the X-axis power transmission antenna with a desired intensity in a desired orientation.

Control of turning on and off of the switches to generate a magnetic field for the Y axis and a magnetic field for the Z axis is similar to the control for the X-axis and can be achieved simply by replacing the components used for the X-axis with the components for the Y- and Z-axes, as in the first embodiment. Therefore description of operations for the Y- and Z-axes will be omitted.

In this way, since the switches 45, 55 and 65 are provided at the power transmission antennas 244, 254 and 264 of the three axes and can be turned on and off by the controller 70 to place only a power transmission antenna of an axis from which a magnetic filed is to be generated in a resonant state and to place the other power transmission antennas of the other two axes in a nonresonant state in the present embodiment, the magnitude of an electric current to pass through each power transmission coil can be stably controlled and the intensity and orientation of the magnetic field generated from the power transmission coil can be properly controlled. Accordingly, electric power can be efficiently supplied to the in-vivo information acquiring device. Furthermore, an induced electromotive force can be inhibited from being generated between power transmission coils and therefore useless electric power is eliminated and energy savings can be achieved.

### (Fourth embodiment)

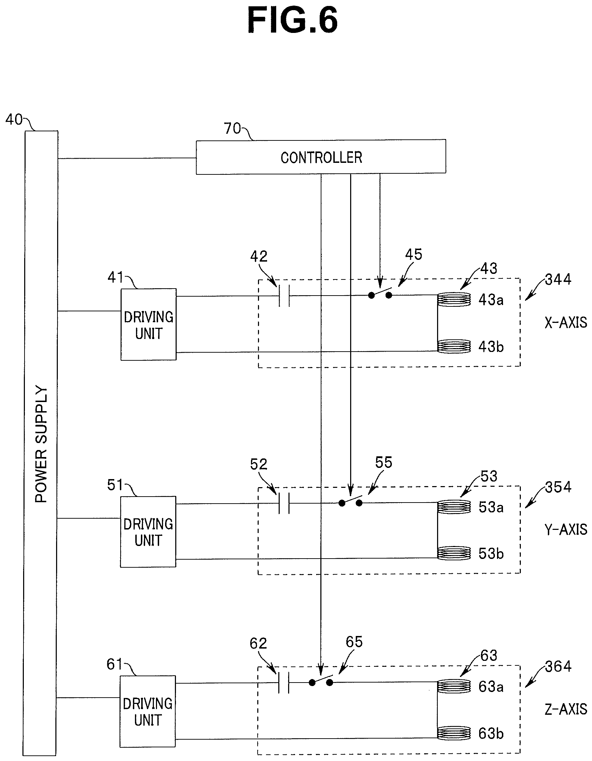

A wireless power feeding system according to a fourth embodiment of the present invention will be described with reference to Fig. 6 in detail. Fig. 6 is a schematic diagram illustrating a configuration of the wireless power feeding system according to the fourth embodiment of the present invention.

The configuration of the wireless power feeding system of the present embodiment is the same as the wireless power feeding system of the first embodiment described with reference to Fig. 1 with the only difference being the circuit configuration of power transmission antennas 344, 354 and 364 corresponding to the X-, Y- and Z axes, respectively. Therefore, only the circuit configuration of the power transmission antennas 344, 354 and 364 will be described here and the same components as those of the first embodiment are given the same reference symbols and description of the same components will be omitted.

The X-, Y- and Z-axis power transmission antennas 344, 354 and 364 have the same circuit configuration. Therefore only the X-axis power transmission antenna 344 will be described here and description of the Y- and Z-axis power transmission antennas 354 and 364 will be omitted.

In the first embodiment, the switch 45 is connected to the ends of the capacitor 42 in the power transmission antenna 44 including a series resonance circuit in which the capacitor 42 and the power transmission coil 43 are connected in series. The present embodiment differs from the first embodiment in that a switch 45 is connected between a capacitor 42 and a power transmission coil 43 in a power transmission antenna 344 including a series resonance circuit in which the capacitor 42 and the power transmission coil 43 are connected in series.

The switch 45 turns on and off the connection between the capacitor 42 and the power transmission coil 43 according to a control signal sent from a controller 70. Switches 55 and 65 are connected between a capacitor 52 and a power transmission coil 53 of the Y-axis and between a capacitor 62 and a power transmission coil 63 of the Z-axis, respectively, as in the power transmission antenna 344.

Specifically, when a magnetic field is to be generated along the X-axis, the switch 45 is turned on. At the same time, the switches 55 and 65 for the Y- and Z-axes are turned off. By controlling and turning on and off the switches 45, 55 and 65 in this way, the X-axis power transmission antenna 344 is placed in a resonant state and the Y-axis power transmission antenna 354 and the Z-axis power transmission antenna 364 are placed in a nonresonant state. Accordingly, a magnetic field can be generated only from the X-axis power transmission antenna with a desired intensity in a desired orientation.

Control of turning on and off of the switches to generate a magnetic field for the Y axis and a magnetic field for the Z axis is similar to the control for the X-axis and can be achieved simply by replacing the components used for the X-axis with the components for the Y- and Z-axes, as in the first embodiment. Therefore description of operations for the Y- and Z-axes will be omitted.

In this way, since the switches 45, 55 and 65 are provided at the power transmission antennas 344, 354 and 364 of the three axes and can be turned on and off by the controller 70 to place only a power transmission antenna of an axis from which a magnetic filed is to be generated in a resonant state and to place the other power transmission antennas of the other two axes in a nonresonant state in the present embodiment, the magnitude of an electric current to pass through each power transmission coil can be stably controlled and the intensity and orientation of the magnetic field generated from the power transmission coil can be properly controlled. Accordingly, electric power can be efficiently supplied to the in-vivo information acquiring device. Furthermore, an induced electromotive force can be inhibited from being generated between power transmission coils and therefore useless electric power is eliminated and energy savings can be achieved.

### (Fifth embodiment)

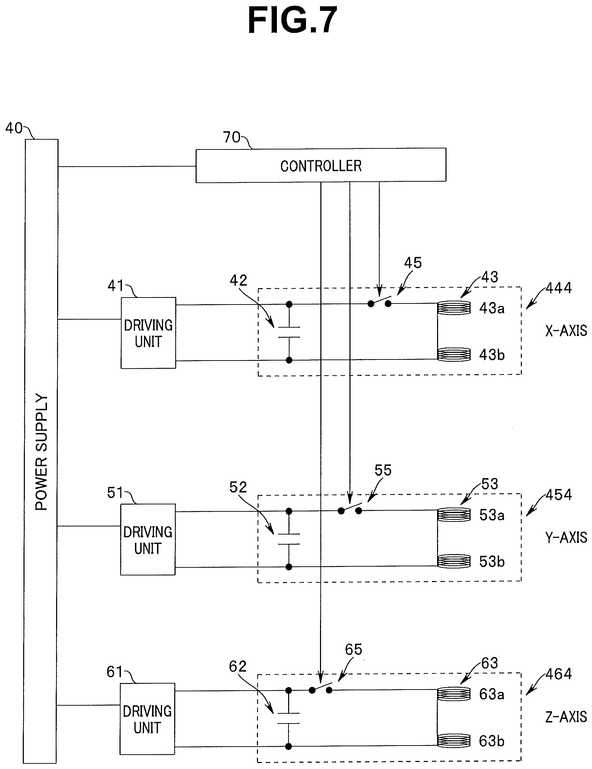

A wireless power feeding system according to a fifth embodiment of the present invention will be described with reference to Fig. 7 in detail. Fig. 7 is a schematic diagram illustrating a configuration of the wireless power feeding system according to the fifth embodiment of the present invention.

The configuration of the wireless power feeding system of the present embodiment is the same as the wireless power feeding system of the first embodiment described with reference to Fig. 1 with the only difference being the circuit configuration of power transmission antennas 444, 454 and 464 corresponding to the X-, Y- and Z axes, respectively. Therefore, only the circuit configuration of the power transmission antennas 444, 454 and 464 will be described here and the same components as those of the first embodiment are given the same reference symbols and description of the same components will be omitted.

The X-, Y- and Z-axis power transmission antennas 444, 454 and 464 have the same circuit configuration. Therefore only the X-axis power transmission antenna 444 will be described here and description of the Y- and Z-axis power transmission antennas 454 and 464 will be omitted.

In the first embodiment, the switch 45 is connected to the ends of the capacitor 42 in the power transmission antenna 44 including a series resonance circuit in which the capacitor 42 and the power transmission coil 43 are connected in series. The present embodiment differs from the first embodiment in that a switch 45 is connected to at least one of two connection points between a capacitor 42 and a power transmission coil 43 in a power transmission antenna 444 including a parallel resonance circuit in which the capacitor 42 and the power transmission coil 43 are connected in parallel.

The switch 45 turns on and off the connection between the capacitor 42 and the power transmission coil 43 according to a control signal sent from the controller 70. As in the power transmission antenna 444, a switch 55 is connected to at least one of two connection points between a capacitor 52 and a power transmission coil 53 of the Y axis and a switch 65 is connected to at least one of two connection points between a capacitor 62 and a power transmission coil 63 of the Z-axis.

Specifically, when a magnetic field is to be generated along the X-axis, the switch 45 is turned on. At the same time, the switches 55 and 65 for the Y- and Z-axes are turned off. By controlling and turning on and off the switches 45, 55 and 65 in this way, the X-axis power transmission antenna 444 is placed in a resonant state and the Y-axis power transmission antenna 454 and the Z-axis power transmission antenna 464 are placed in a nonresonant state. Accordingly, a magnetic field can be generated only from the X-axis power transmission antenna with a desired intensity in a desired orientation.

Control of turning on and off of the switches to generate a magnetic field for the Y axis and a magnetic field for the Z axis is similar to the control for the X-axis and can be achieved simply by replacing the components used for the X-axis with the components for the Y- and Z-axes. Therefore description of operations for the Y-and Z-axes will be omitted.

In this way, since the switches 45, 55 and 65 are provided at the power transmission antennas 444, 454 and 464 of the three axes and can be turned on and off by the controller 70 to place only a power transmission antenna of an axis from which a magnetic filed is to be generated in a resonant state and to place the other power transmission antennas of the other two axes in a nonresonant state in the present embodiment, the magnitude of an electric current to pass through each power transmission coil can be stably controlled and the intensity and orientation of the magnetic field generated from the power transmission coil can be properly controlled. Accordingly, electric power can be efficiently supplied to the in-vivo information acquiring device. Furthermore, an induced electromotive force can be inhibited from being generated between power transmission coils and therefore useless electric power is eliminated and energy savings can be achieved.

### (Sixth embodiment)

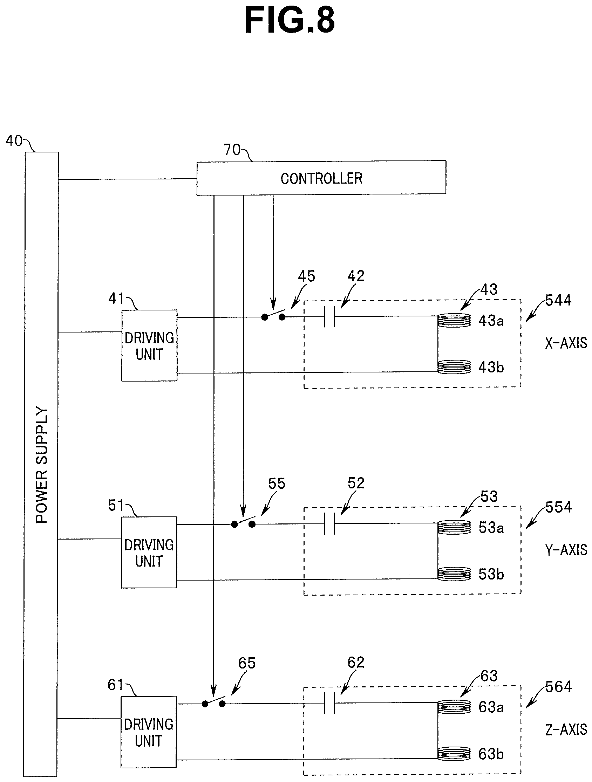

A wireless power feeding system according to a sixth embodiment of the present invention will be described with reference to Fig. 8 in detail. Fig. 8 is a schematic diagram illustrating a configuration of the wireless power feeding system according to the sixth embodiment of the present invention.

The configuration of the wireless power feeding system of the present embodiment is the same as the wireless power feeding system of the first embodiment described with reference to Fig. 1 with the only difference being the circuit configuration of power transmission antennas 544, 554 and 564 corresponding to the X-, Y- and Z axes, respectively. Therefore, only the circuit configuration of the power transmission antennas 544, 554 and 564 will be described here and the same components as those of the first embodiment are given the same reference symbols and description of the same components will be omitted.

The X-, Y- and Z-axis power transmission antennas 544, 554 and 564 have the same circuit configuration. Therefore only the X-axis power transmission antenna 544 will be described here and description of the Y- and Z-axis power transmission antennas 554 and 564 will be omitted.

In the first embodiment, the switch 45 is connected to the ends of the capacitor 42 in the power transmission antenna 44 including a series resonance circuit in which the capacitor 42 and the power transmission coil 43 are connected in series. The present embodiment differs from the first embodiment in that a switch 45 is connected between a driving unit 41 and a capacitor 42 in a power transmission antenna 544 including a series resonance circuit in which the capacitor 42 and the power transmission coil 43 are connected in series.

The switch 45 turns on and off the connection between the driving unit 41 and the capacitor 42 according to a control signal sent from the controller 70. As in the power transmission antenna 544, a switch 55 is connected between a driving unit 51 and a capacitor 52 of the Y-axis and a switch 65 is connected between a driving unit 61 and a capacitor 62 of the Z-axis.

Specifically, when a magnetic field is to be generated along the X-axis, the switch 45 is turned on. At the same time, the switches 55 and 65 for the Y- and Z-axes are turned off. By controlling and turning on and off the switches 45, 55 and 65 in this way, the X-axis power transmission antenna 544 is placed in a resonant state and the Y-axis power transmission antenna 554 and the Z-axis power transmission antenna 564 are placed in a nonresonant state. Accordingly, a magnetic field can be generated only from the X-axis power transmission antenna with a desired intensity in a desired orientation.

Control of turning on and off of the switches to generate a magnetic field for the Y axis and a magnetic field for the Z axis is similar to the control for the X-axis and can be achieved simply by replacing the components used for the X-axis with the components for the Y- and Z-axes. Therefore description of operations for the Y-and Z-axes will be omitted.

In this way, since the switches 45, 55 and 65 are provided at the power transmission antennas 544, 554 and 564 of the three axes and can be turned on and off by the controller 70 to place only a power transmission antenna of an axis from which a magnetic filed is to be generated in a resonant state and to place the other power transmission antennas of the other two axes in a nonresonant state in the present embodiment, the magnitude of an electric current to pass through each power transmission coil can be stably controlled and the intensity and orientation of the magnetic field generated from the power transmission coil can be properly controlled. Accordingly, electric power can be efficiently supplied to the in-vivo information acquiring device. Furthermore, an induced electromotive force can be inhibited from being generated between power transmission coils and therefore useless electric power is eliminated and energy savings can be achieved.

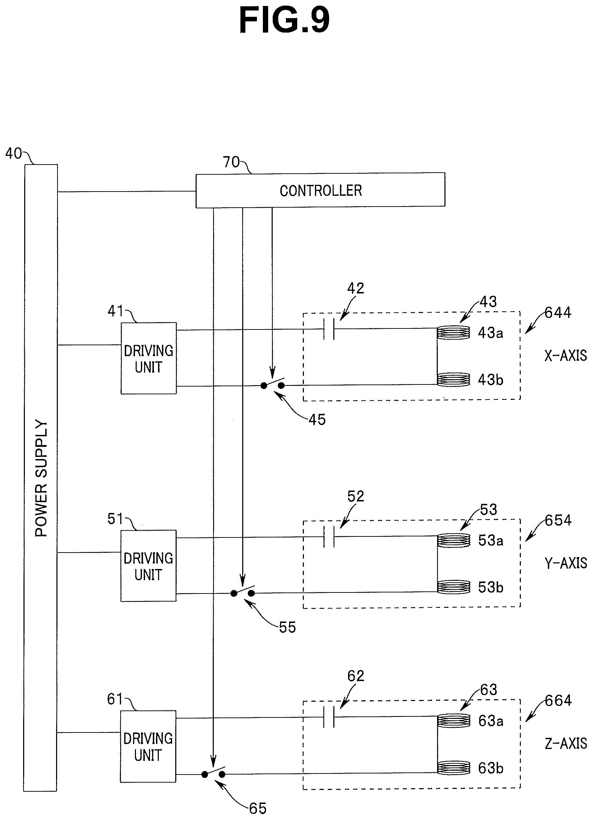

While each switch 45, 55, 65 is provided between the driving unit 41, 51, 61, and the capacitor 42, 52, 62 in the present embodiment, the circuit configuration of the power transmission antennas may be modified as illustrated in Fig. 9 because it is essential only that switching control of (on and off of) the electrical connection between the driving unit 41, 51, 61 and the power transmission antenna 544, 554, 564 can be accomplished with a switch 45, 55, 65. Fig. 9 is a schematic diagram illustrating a variation of the wireless power feeding system according to the sixth embodiment of the present invention.

As illustrated in Fig. 9, the switch 45, 55, 65 may be provided between the driving unit 41, 51, 61 and the power transmission coil 43, 53, 63.

As has been described above, according to any of the embodiments described above, there can be provided a wireless power feeding system capable of efficiently supplying electric power by stably controlling the magnitude of an electric current passing through a power transmission coil and appropriately controlling the intensity and orientation of a magnetic field generated by the power transmission coil.

While the six embodiments have been described with respect to power transmission antennas or three axes, X, Y and Z, the present invention is applicable to power transmission antennas of more than one axis.

While wireless power feeding systems of the present invention have been described with respect to a capsule endoscope as an example of the in-vivo information acquiring device, the present invention is not limited to the embodiments described above.

For example, the present invention is also applicable to a physiological sensor or a medical device as an in-vivo information acquiring system.

### Industrial Applicability

It will be understood that the wireless power feeding system of the present invention is applicable to a wide variety of apparatuses that wirelessly supply electric power, in addition to in-vivo information acquiring devices mentioned above.

## Claims

1. A wireless power feeding system comprising:
a plurality of power transmission antennas (44, 54, 64), each comprising a resonance circuit including a power transmission coil (43, 53, 63) and a capacitor (42, 52, 62) located so as to generate a magnetic field in a desired direction, and a switch (45, 55, 65);
a controller (70) adapted to control a resonant state of the resonance circuit of each of the plurality of power transmission antennas (44, 54, 64);
a plurality of driving units (41, 51, 61) adapted to apply an AC voltage to the plurality of power transmission antennas (44, 54, 64) to drive each of the plurality of power transmission antennas (44, 54, 64); and
a power supply unit (40) adapted to supply a voltage to the driving units (41, 51, 61); wherein
the controller (70) is adapted to control, among the plurality of power transmission antennas (44, 54, 64), one power transmission antenna from which a magnetic field is to be generated to place the resonance circuit of the one power transmission antenna in a resonant state,
the controller (70) is adapted to control, among the plurality of power transmission antennas (44, 54, 64), all of the at least one remaining power transmission antenna from which a magnetic field is not to be generated to place the resonance circuit of the all of the at least one remaining power transmission antenna in a nonresonant state; and
the controller (70) is adapted to turn on and off the switch (45, 55, 65) of each of the plurality of power transmission antennas (44, 54, 64) to control the resonant state of each of the plurality of power transmission antennas (44, 54, 64);
**characterized in that**
the switch (45, 55, 65) is connected to both ends of the capacitor (42, 52, 62) and/or of the power transmission coil (43, 53, 63).

2. A wireless power feeding system comprising:
a plurality of power transmission antennas (44, 54, 64), each comprising a resonance circuit including a power transmission coil (43, 53, 63) and a capacitor (42, 52, 62) located so as to generate a magnetic field in a desired direction, and a switch (45, 55, 65);
a controller (70) adapted to control a resonant state of the resonance circuit of each of the plurality of power transmission antennas (44, 54, 64);
a plurality of driving units (41, 51, 61) adapted to apply an AC voltage to the plurality of power transmission antennas (44, 54, 64) to drive each of the plurality of power transmission antennas (44, 54, 64); and
a power supply unit (40) adapted to supply a voltage to the driving units (41, 51, 61);
wherein
the controller (70) is adapted to control, among the plurality of power transmission antennas (44, 54, 64), one power transmission antenna from which a magnetic field is to be generated to place the resonance circuit of the one power transmission antenna in a resonant state,
the controller (70) is adapted to control, among the plurality of power transmission antennas (44, 54, 64), all of the at least one remaining power transmission antenna from which a magnetic field is not to be generated to place the resonance circuit of the all of the at least one remaining power transmission antenna in a nonresonant state;
the controller (70) is adapted to turn on and off the switch (45, 55, 65) of each of the plurality of power transmission antennas (44, 54, 64) to control the resonant state of each of the plurality of power transmission antennas (44, 54, 64);
**characterized in that**
the switch (45, 55, 65) is connected between the capacitor (42, 52, 62) and the power transmission coil (43, 53, 63).

3. A wireless power feeding system comprising:
a plurality of power transmission antennas (44, 54, 64), each comprising a resonance circuit including a power transmission coil (43, 53, 63) and a capacitor (42, 52, 62) located so as to generate a magnetic field in a desired direction, and a switch (45, 55, 65);
a controller (70) adapted to control a resonant state of the resonance circuit of each of the plurality of power transmission antennas (44, 54, 64);
a plurality of driving units (41, 51, 61) adapted to apply an AC voltage to the plurality of power transmission antennas (44, 54, 64) to drive each of the plurality of power transmission antennas (44, 54, 64); and
a power supply unit (40) adapted to supply a voltage to the driving units (41, 51, 61);
wherein
the controller (70) is adapted to control, among the plurality of power transmission antennas (44, 54, 64), one power transmission antenna from which a magnetic field is to be generated to place the resonance circuit of the one power transmission antenna in a resonant state,
the controller (70) is adapted to control, among the plurality of power transmission antennas (44, 54, 64), all of the at least one remaining power transmission antenna from which a magnetic field is not to be generated to place the resonance circuit of the all of the at least one remaining power transmission antenna in a nonresonant state;
the controller (70) is adapted to turn on and off the switch (45, 55, 65) of each of the plurality of power transmission antennas (44, 54, 64) to control the resonant state of each of the plurality of power transmission antennas (44, 54, 64);
**characterized in that**
the switch (45, 55, 65) is connected between the driving unit and the capacitor (42, 52, 62) or power transmission coil (43, 53, 63).

4. The wireless power feeding system according to any one of claims 1 to 3, wherein the plurality of power transmission antennas (44, 54, 64) include three power transmission antennas (44, 54, 64) located so as to generate a magnetic field parallel to each axis of a predetermined three-dimensional Cartesian coordinate system.

5. The wireless power feeding system according to claim 4, wherein the controller (70) is adapted to control, among the three power transmission antennas (44, 54, 64), one power transmission antenna, from which a magnetic filed is to be generated, to place the resonance circuit of the one power transmission antenna in the resonant state, and the controller (70) is adapted to control the other two of the three power transmission antennas, from which a magnetic field is not to be generated, to place the resonance circuits of the other two power transmission antennas in the nonresonant state.

6. The wireless power feeding system according to any one of claims 1 to 5, wherein the power transmission coil (43, 53, 63) and the capacitor (42, 52, 62) are connected in series.

7. The wireless power feeding system according to any one of claims 1 to 5, wherein the power transmission coil (43, 53, 63) and the capacitor (42, 52, 62) are connected in parallel.

8. The wireless power feeding system according to claim 6 or 7, wherein the switch (45, 55, 65) is connected in parallel with the capacitor (42, 52, 62).

9. The wireless power feeding system according to claim 6, wherein the switch (45, 55, 65) is connected in parallel with the power transmission coil (43, 53, 63).

10. The wireless power feeding system according to any one of claims 1 to 5, placing the power transmission antenna in a resonant state and transmitting electric power to an in-vivo information acquiring device (71) comprising a power receiving antenna including a power receiving coil for receiving electric power wirelessly transmitted.

11. The wireless power feeding system according to claim 10, wherein the in-vivo information acquiring device is a capsule endoscope.

## Patentansprüche

1. Drahtloses Energiespeisungssystem, das aufweist:
mehrere Energieübertragungsantennen (44, 54, 64), die jeweils eine Resonanzschaltung, die eine Energieübertragungsspule (43, 53, 63) und einen Kondensator (42, 52, 62) enthält und die derart angeordnet ist, dass sie ein magnetisches Feld in einer gewünschten Richtung erzeugt, und einen Schalter (45, 55, 65) aufweisen;
eine Steuerung (70), die ausgelegt ist, einen Resonanzzustand der Resonanzschaltung jeder der Energieübertragungsantennen (44, 54, 64) zu steuern;
mehrere Ansteuereinheiten (41, 51, 61), die ausgelegt sind, eine AC-Spannung an jede der Energieübertragungsantennen (44, 54, 64) anzulegen, um die jeweiligen Energieübertragungsantennen (44, 54, 64) anzusteuern; und
eine Energieversorgungseinheit (40), die ausgelegt ist, den Ansteuerschaltungen (41, 51, 61) eine Spannung zuzuführen;
wobei
die Steuerung (70) ausgelegt ist, unter den Energieübertragungsantennen (44, 54, 64) eine Energieübertragungsantenne zu steuern, von der ein magnetisches Feld zu erzeugen ist, um die Resonanzschaltung der einen Energieübertragungsantenne in einen Resonanzzustand zu versetzen,
die Steuerung (70) ausgelegt ist, unter den Energieübertragungsantennen (44, 54, 64) sämtliche der mindestens einen verbleibenden Energieübertragungsantenne zu steuern, von der ein Magnetfeld nicht zu erzeugen ist, um die Resonanzschaltung sämtlicher der mindestens einen verbleibenden Energieübertragungsantenne in einen Nichtresonanzzustand zu versetzen, und
die Steuerung (70) ausgelegt ist, den Schalter (45, 55, 65) jeder der Energieübertragungsantennen (44, 54, 64) ein- und auszuschalten, um den Resonanzzustand der jeweiligen Energieübertragungsantennen (44, 54, 64) zu steuern;
**dadurch gekennzeichnet, dass**
der Schalter (45, 55, 65) mit beiden Enden des Kondensators (42, 52, 62) und/oder der Energieübertragungsspule (43, 53, 63) verbunden ist.

2. Drahtloses Energiespeisungssystem, das aufweist:
mehrere Energieübertragungsantennen (44, 54, 64), die jeweils eine Resonanzschaltung, die eine Energieübertragungsspule (43, 53, 63) und einen Kondensator (42, 52, 62) enthält und die derart angeordnet ist, dass sie ein magnetisches Feld in einer gewünschten Richtung erzeugt, und einen Schalter (45, 55, 65) aufweisen;
eine Steuerung (70), die ausgelegt ist, einen Resonanzzustand der Resonanzschaltung jeder der Energieübertragungsantennen (44, 54, 64) zu steuern;
mehrere Ansteuereinheiten (41, 51, 61), die ausgelegt sind, eine AC-Spannung an jede der Energieübertragungsantennen (44, 54, 64) anzulegen, um die jeweiligen Energieübertragungsantennen (44, 54, 64) anzusteuern; und
eine Energieversorgungseinheit (40), die ausgelegt ist, den Ansteuerschaltungen (41, 51, 61) eine Spannung zuzuführen;
wobei
die Steuerung (70) ausgelegt ist, unter den Energieübertragungsantennen (44, 54, 64) eine Energieübertragungsantenne zu steuern, von der ein magnetisches Feld zu erzeugen ist, um die Resonanzschaltung der einen Energieübertragungsantenne in einen Resonanzzustand zu versetzen,
die Steuerung (70) ausgelegt ist, unter den Energieübertragungsantennen (44, 54, 64) sämtliche der mindestens einen verbleibenden Energieübertragungsantenne zu steuern, von der ein Magnetfeld nicht zu erzeugen ist, um die Resonanzschaltung sämtlicher der mindestens einen verbleibenden Energieübertragungsantenne in einen Nichtresonanzzustand zu versetzen, und
die Steuerung (70) ausgelegt ist, den Schalter (45, 55, 65) jeder der Energieübertragungsantennen (44, 54, 64) ein- und auszuschalten, um den Resonanzzustand der jeweiligen Energieübertragungsantennen (44, 54, 64) zu steuern;
**dadurch gekennzeichnet, dass**
der Schalter (45, 55, 65) zwischen den Kondensator (42, 52, 62) und die Energieübertragungsspule (43, 53, 63) geschaltet ist.

3. Drahtloses Energiespeisungssystem, das aufweist:
mehrere Energieübertragungsantennen (44, 54, 64), die jeweils eine Resonanzschaltung, die eine Energieübertragungsspule (43, 53, 63) und einen Kondensator (42, 52, 62) enthält und die derart angeordnet ist, dass sie ein magnetisches Feld in einer gewünschten Richtung erzeugt, und einen Schalter (45, 55, 65) aufweise;
eine Steuerung (70), die ausgelegt ist, einen Resonanzzustand der Resonanzschaltung jeder der Energieübertragungsantennen (44, 54, 64) zu steuern;
mehrere Ansteuereinheiten (41, 51, 61), die ausgelegt sind, eine AC-Spannung an jede der Energieübertragungsantennen (44, 54, 64) anzulegen, um die jeweiligen Energieübertragungsantennen (44, 54, 64) anzusteuern; und
eine Energieversorgungseinheit (40), die ausgelegt ist, den Ansteuerschaltungen (41, 51, 61) eine Spannung zuzuführen;
wobei
die Steuerung (70) ausgelegt ist, unter den Energieübertragungsantennen (44, 54, 64) eine Energieübertragungsantenne zu steuern, von der ein magnetisches Feld zu erzeugen ist, um die Resonanzschaltung der einen Energieübertragungsantenne in einen Resonanzzustand zu versetzen,
die Steuerung (70) ausgelegt ist, unter den Energieübertragungsantennen (44, 54, 64) sämtliche der mindestens einen verbleibenden Energieübertragungsantenne zu steuern, von der ein Magnetfeld nicht zu erzeugen ist, um die Resonanzschaltung sämtlicher der mindestens einen verbleibenden Energieübertragungsantenne in einen Nichtresonanzzustand zu versetzen, und
die Steuerung (70) ausgelegt ist, den Schalter (45, 55, 65) jeder der Energieübertragungsantennen (44, 54, 64) ein- und auszuschalten, um den Resonanzzustand der jeweiligen Energieübertragungsantennen (44, 54, 64) zu steuern;
**dadurch gekennzeichnet, dass**
der Schalter (45, 55, 65) zwischen die Ansteuereinheit und den Kondensator (42, 52, 62) oder die Energieübertragungsspule (43, 53, 63) geschaltet ist.

4. Drahtloses Energiespeisungssystem nach einem der Ansprüche 1 bis 3, wobei die Energieübertragungsantennen (44, 54, 64) drei Energieübertragungsantennen (44, 54, 64) enthalten, die derart angeordnet sind, dass sie ein magnetisches Feld parallel zu jeder Achse eines vorbestimmten dreidimensionalen kartesischen Koordinatensystems erzeugen.

5. Drahtloses Energiespeisungssystem nach Anspruch 4, wobei die Steuerung (70) ausgelegt ist, unter den drei Energieübertragungsantennen (44, 54, 64) eine Energieübertragungsantenne zu steuern, von der ein magnetisches Feld zu erzeugen ist, um die Resonanzschaltung der einen Energieübertragungsantenne in den Resonanzzustand zu versetzen, und
die Steuerung (70) ausgelegt ist, die anderen beiden der drei Energieübertragungsantennen zu steuern, von denen ein Magnetfeld nicht zu erzeugen ist, um die Resonanzschaltungen der beiden anderen Energieübertragungsantennen in den Nichtresonanzzustand zu versetzen.

6. Drahtloses Energiespeisungssystem nach einem der Ansprüche 1 bis 5, wobei die Energieübertragungsspule (43, 53, 63) und der Kondensator (42, 52, 62) in Serie geschaltet sind.

7. Drahtloses Energiespeisungssystem nach einem der Ansprüche 1 bis 5, wobei die Energieübertragungsspule (43, 53, 63) und der Kondensator (42, 52, 62) parallel geschaltet sind.

8. Drahtloses Energiespeisungssystem nach Anspruch 6 oder 7, wobei der Schalter (45, 55, 65) parallel zu dem Kondensator (42, 52, 62) geschaltet ist.

9. Drahtloses Energiespeisungssystem nach Anspruch 6, wobei der Schalter (45, 55, 65) parallel zu der Energieübertragungsspule (43, 53, 63) geschaltet ist.

10. Drahtloses Energiespeisungssystem nach einem der Ansprüche 1 bis 5, das die Energieübertragungsantenne in einen Resonanzzustand versetzt und elektrische Energie an eine In-vivo-Informationsbeschaffungsvorrichtung (71) überträgt, die eine Energieempfangsantenne aufweist, die eine Energieempfangsspule zum Empfangen von elektrischer Energie, die drahtlos übertragen wird, enthält.

11. Drahtloses Energiespeisungssystem nach Anspruch 10, wobei die In-vivo-Informationsbeschaffungsvorrichtung ein Kapselendoskop ist.

## Revendications

1. Système d'alimentation électrique sans fil, comprenant:
une pluralité d'antennes de transmission de puissance (44, 54, 64), comprenant chacune un circuit de résonance comprenant une bobine de transmission de puissance (43, 53, 63) et un condensateur (42, 52, 62) disposé de manière à générer un champ magnétique dans une direction souhaitée, et un commutateur (45, 55, 65) ;
un dispositif de commande (70) approprié pour commander un état résonant du circuit de résonance de chacune de la pluralité d'antennes de transmission de puissance (44, 54, 64) ;
une pluralité d'unités d'activation (41, 51, 61) appropriés pour appliquer une tension de courant alternatif à la pluralité d'antennes de transmission de puissance (44, 54, 64) afin d'activer chacune de pluralité d'antennes de transmission de puissance (44, 54, 64) ; et
une unité d'alimentation électrique (40) appropriée pour fournir une tension aux unités d'activation (41, 51, 61),
dans lequel:
le dispositif de commande (70) est approprié pour commander, parmi la pluralité d'antennes de transmission de puissance (44, 54, 64), une première antenne de transmission de puissance à partir de laquelle un champ magnétique doit être généré afin de placer le circuit de résonance de ladite une première antenne de transmission de puissance dans un état résonant,
le dispositif de commande (70) est approprié pour commander, parmi la pluralité d'antennes de transmission de puissance (44, 54, 64), chacune de ladite au moins une antenne de transmission de puissance restante à partir de laquelle un champ magnétique ne doit pas être généré afin de placer le circuit de résonance de chacune de ladite au moins une première antenne de transmission de puissance restante dans un état non résonant ; et
le dispositif de commande (70) est approprié pour activer et désactiver le commutateur (45, 55, 65) de chacune de la pluralité d'antennes de transmission de puissance (44, 54, 64) afin de commander l'état résonant de chacune de la pluralité d'antennes de transmission de puissance (44, 54, 64)
**caractérisé en ce que** le commutateur (45, 55, 65) est connecté aux deux extrémités du condensateur (42, 52, 62) et/ou de la bobine de transmission de puissance (43, 53, 63) .

2. Système d'alimentation électrique sans fil, comprenant:
une pluralité d'antennes de transmission de puissance (44, 54, 64), comprenant chacune un circuit de résonance comprenant une bobine de transmission de puissance (43, 53, 63) et un condensateur (42, 52, 62) disposé manière à générer un champ magnétique dans une direction souhaitée et un commutateur (45, 55, 65) ;
un dispositif de commande (70) approprié pour commander un état résonant du circuit de résonance de chacune de la pluralité d'antennes de transmission de puissance (44, 54, 64) ;
une pluralité d'unités d'activation (41, 51, 61) appropriées pour appliquer une tension de courant alternatif à la pluralité d'antennes de transmission de puissance (44, 54, 64) afin d'activer chacune de pluralité d'antennes de transmission de puissance (44, 54, 64) et
une unité d'alimentation électrique (40) appropriée pour fournir une tension aux unités d'activation (41, 51, 61),
dans lequel:
le dispositif de commande (70) est approprié pour commander, parmi la pluralité d'antennes de transmission de puissance (44, 54, 64), une antenne de transmission de puissance à partir de laquelle un champ magnétique doit être généré afin de placer le circuit de résonance de ladite une antenne de transmission de puissance dans un état résonant,
le dispositif de commande (70) est approprié pour commander, parmi la pluralité d'antennes de transmission de puissance (44, 54, 64), chacune de ladite au moins une antenne de transmission de puissance restante à partir de laquelle un champ magnétique ne doit pas être généré afin de placer le circuit de résonance de chacune de ladite au moins une première antenne de transmission de puissance restante dans un état non résonant et
le dispositif de commande (70) est approprié pour activer et désactiver le commutateur (45, 55, 65) de chacune de la pluralité d'antennes de transmission de puissance (44, 54, 64) afin de commander l'état résonant de chacune de la pluralité d'antennes de transmission de puissance (44, 54, 64)
**caractérisé en ce que** le commutateur (45, 55, 65) est connecté entre le condensateur (42, 52, 62) et la bobine de transmission de puissance (53, 53, 63).

3. Système d'alimentation électrique sans fil, comprenant:
une pluralité d'antennes de transmission de puissance (44, 54, 64), comprenant chacune un circuit de résonance comprenant une bobine de transmission de puissance (43, 53, 63) et un condensateur (42, 52, 62) disposé manière à générer un champ magnétique dans une direction souhaitée, et un commutateur ;
une pluralité d'unités d'activation (41, 51, 61) appropriées pour appliquer une tension de courant alternatif à la pluralité d'antennes de transmission de puissance (44, 54, 64) afin d'activer chacune de pluralité d'antennes de transmission de puissance (44, 54, 64) et
une unité d'alimentation électrique (40) approprié pour fournir une tension aux unités d'activation (41, 51, 61),
dans lequel:
le dispositif de commande (70) est approprié pour commander, parmi la pluralité d'antennes de transmission de puissance (44, 54, 64), une antenne de transmission de puissance à partir de laquelle un champ magnétique doit être généré afin de placer le circuit de résonance de ladite une antenne de transmission de puissance dans un état résonant,
le dispositif de commande (70) est approprié pour commander, parmi la pluralité d'antennes de transmission de puissance (44, 54, 64), chacune de ladite au moins une antenne de transmission de puissance restante à partir de laquelle un champ magnétique ne doit pas être généré afin de placer le circuit de résonance de chacune de ladite au moins une antenne de transmission de puissance restante dans un état non résonant ; et
le dispositif de commande (70) est approprié pour activer et désactiver le commutateur (45, 55, 65) de chacune de la pluralité d'antennes de transmission de puissance (44, 54, 64) afin de commander l'état résonant de chacune de la pluralité d'antennes de transmission de puissance (44, 54, 64) ;
**caractérisé en ce que** le commutateur (45, 55, 65) est connecté entre l'unité d'activation et le condensateur (42, 52, 62) ou la bobine de transmission de puissance (53, 53, 63) .

4. Système d'alimentation électrique sans fil selon l'une quelconque des revendications 1 à 3, dans lequel la pluralité d'antennes de transmission de puissance (44, 54, 64) comprend trois antennes de transmission de puissance (44, 54, 64) disposées de manière à générer un champ magnétique parallèle à chaque axe d'un système de coordonnées cartésiennes tridimensionnelles prédéterminé.

5. Système d'alimentation électrique sans fil selon la revendication 4, dans lequel le dispositif de commande (70) est approprié pour commander, parmi les trois antennes de transmission de puissance (44, 54, 64), une antenne de transmission de puissance, à partir de laquelle un champ magnétique est généré, afin de placer le circuit de résonance de ladite première antenne de transmission de puissance dans l'état résonant et
le dispositif de commande (70) est approprié pour commander les deux autres des trois antennes de transmission de puissance, à partir desquelles un champ magnétique n'est pas généré, afin de placer les circuits de résonance des deux autres antennes de transmission de puissance dans l'état non résonant.

6. Système d'alimentation électrique sans fil selon l'une quelconque des revendications 1 à 5, dans lequel la bobine de transmission de puissance (43, 53, 63) et le condensateur (42, 52, 62) sont raccordés en série.

7. Système d'alimentation électrique sans fil selon l'une quelconque des revendications 1 à 5, dans lequel la bobine de transmission de puissance (43, 53, 63) et le condensateur (42, 52, 62) sont raccordés en parallèle.

8. Système d'alimentation électrique sans fil selon la revendication 6 ou 7, dans lequel le commutateur (45, 55, 65) est raccordé en parallèle avec le condensateur (42, 52, 62).

9. Système d'alimentation électrique sans fil selon la revendication 6, dans lequel le commutateur (45, 55, 65) est raccordé en parallèle avec la bobine de transmission de puissance (43, 53, 63).

10. Système d'alimentation électrique sans fil selon l'une quelconque des revendications 1 à 5 plaçant l'antenne de transmission de puissance dans un état résonant et transmettant la puissance électrique à un dispositif d'acquisition d'informations in vivo (71) qui comprend une antenne de réception de puissance incluant une bobine de réception de puissance pour recevoir la puissance électrique transmise sans fil.

11. Système d'alimentation électrique sans fil selon la revendication 10, dans lequel le dispositif d'acquisition d'informations in vivo est une capsule endoscopique.
